# EUROPÄISCHE PATENTANMELDUNG

(11) **EP 0 855 385 A1**
(43) Veröffentlichungstag der Anmeldung: **29.07.1998**
(21) Anmeldenummer: 98100344.5
(22) Anmeldetag: 10.01.1998
(51) Int. Cl.: C07C 213/06, C07C 213/00, C07C 217/84

(54) **Verfahren zur Herstellung von p-Haloalkoxyanilinen**

(30) Priorität: 23.01.1997 DE 19702207
(71) Anmelder: BAYER AG, 51368 Leverkusen (DE)
(72) Erfinder: Mais, Franz-Josef, Dr., 40591 Düsseldorf (DE); Marhold, Albrecht, Dr., 51373 Leverkusen (DE); Steffan, Guido, Dr., 51519 Odenthal (DE)

(57) **Zusammenfassung**

p-Haloalkoxyaniline werden aus den entsprechenden Nitrobenzolen durch eine katalytische Hydroxylierung in Gegenwart eines wäßrig-sauren Reaktionsmediums unter Bildung eines entsprechenden Aminophenols und Umsetzung dieses Aminophenols mit einem halogenierten Olefin in Gegenwart von Wasser und einer katalytischen Menge an Base erhalten.

## Beschreibung

Die vorliegende Erfindung betrifft ein Verfahren zur Herstellung von p-Haloalkoxyanilinen durch katalytische Hydroxylierung von Nitrobenzolen und anschließender Veretherung der erhaltenen OH-Gruppe durch Umsetzung mit halogenierten Olefinen.

p-Haloalkoxyaniline sind wichtige Zwischenprodukte zur Herstellung von insektiziden Wirkstoffen (siehe z.B. EP-OS 179 022, DE-OS 36 27 161, EP-OS 221 847, EP-OS 235 089 und EP-OS 343 110). Die Herstellung dieser Aniline erfolgt üblicherweise durch Umsetzung von halogenierten Olefinen mit p-Nitrophenolderivaten und anschließender Hydrierung der Nitrogruppe (siehe z.B. EP-OS 179 022 und EP-OS 235 089), durch Umsetzung halogenierter Olefine mit N-acylierten p-Aminophenolen und anschließender Abspaltung der N-Acylgruppe (siehe z.B. EP-OS 179 022 und EP-OS 235 089), durch Umsetzung der Salze von p-Aminophenolen mit halogenierten Olefinen (siehe z.B EP-OS 179 022 und US-PS 4 518 804) oder durch Umsetzung von p-Fluornitrobenzolderivaten mit Halogenalkoholen (siehe z.B. EP-OS 235 089) und anschließender katalytischer Hydrierung der Nitrogruppe.

Nachteilig bei diesen Verfahren ist die Anzahl der Verfahrensstufen, insbesondere unter Einbeziehung der Herstellung der entsprechenden Ausgangsstoffe. So müssen beispielsweise p-Nitrophenolderivate in einem vorgelagerten Schritt durch Nitrierung eines Phenols hergestellt werden, und nach der Umsetzung mit halogenierten Olefinen ist ein weiterer Schritt zur Reduktion der Nitrogruppe notwendig. Von großem Nachteil ist auch, daß bei der Nitrierung auch stellungsisomere Verbindungen gebildet werden, die abgetrennt werden müssen. Bei der Umsetzung von N-acylierten Aminophenolen bedeutet die Einführung und Abspaltung der Schutzgruppe einen deutlich erhöhten Verfahrensaufwand. Auch die Herstellung der Aminophenolsalze durch Nitrierung eines Phenols und anschließender Hydrierung und Salzbildung ist bezüglich der Anzahl der Verfahrensstufen und der Verfügbarkeit der Edukte ungünstig Dies gilt ebenso für die Umsetzung von p-Fluornitroaromaten mit Halogenalkanolen.

Es besteht daher die Aufgabe, ein Verfahren zur Herstellung von p-Haloalkoxyanilinen zu finden, das mehrstufige und aufwendig Reaktionssequenzen vermeidet und von einfach zugänglichen Edukten ausgeht.

Es wurde nun ein Verfahren zur Herstellung von p-Haloalkoxyanilinen der Formel (I) gefunden 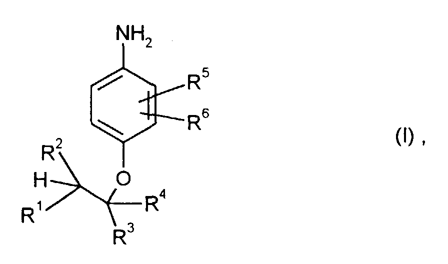 in der
- R¹: Wasserstoff, Halogen oder C₁-C₄-Halogenalkyl und
- R² bis R⁶: unabhängig voneinander jeweils Wasserstoff oder Halogen bedeuten, wobei mindestens einer der Reste R¹ bis R⁴ verschieden von Wasserstoff ist,
das dadurch gekennzeichnet ist, daß man ein Nitrobenzol der Formel (II) 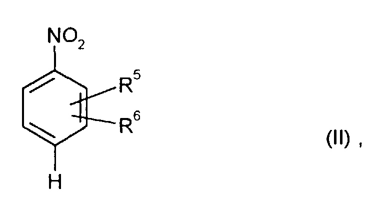 in der
R⁵ und R⁶ die bei Formel (I) angegebene Bedeutung haben,
durch katalytische hydrierende Hydroxylierung in Gegenwart eines wäßrig-sauren Reaktionsmediums in ein freies Aminophenol der Formel (III) umwandelt 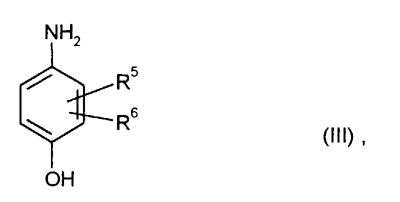 in der
R⁵ und R⁶ die bei Formel (I) angegebene Bedeutung haben,
und dieses Aminophenol mit einem halogenierten Olefin der Formeln (IV) oder (V) 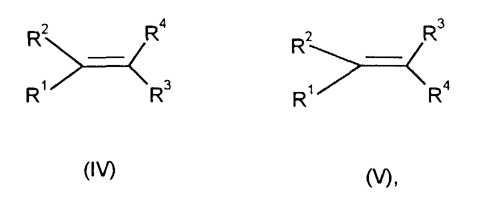 in denen
R¹ bis R⁴ die bei Formel (I) angegebene Bedeutung haben,
in Gegenwart von Wasser und einer katalytischen Menge an Base umsetzt.

Halogen, auch in Halogenalkyl, steht beispielsweise für Fluor, Chlor oder Brom.

Vorzugsweise stehen R¹ für C₁-C₂-Halogenalkyl, Fluor oder Chlor, R² bis R⁴ unabhängig voneinander für Wasserstoff, Fluor oder Chlor und R⁵ und R⁶ unabhängig voneinander für Wasserstoff, Fluor, Chlor oder Brom, wobei mindestens einer der Reste R⁵ und R⁶ nicht Wasserstoff ist.

Besonders bevorzugt stehen R¹ für Perfluor-C₁-C₂-alkyl wie Trifluormethyl oder Pentafluorethyl, R² bis R⁴ unabhängig voneinander für Fluor oder Chlor und R⁵ und R⁶ unabhängig voneinander für Wasserstoff oder Chlor, wobei wenigstens einer der Reste R⁵ und R⁶ für Chlor steht.

Die katalytische hydrierende Hydroxylierung kann z.B. mit Wasserstoff unter Druck bei erhöhter Temperatur und in Gegenwart von Katalysatoren durchgeführt werden.

Das wäßrig-saure Reaktionsmedium kann aus einem Gemisch bestehen, das Wasser und eine starke Säure enthält. Die starke Säure kann anorganisch oder organisch sein. Beispiele sind Schwefelsäure, Salzsäure, Bromwasserstoffsäure, Phosphorsäure, Methansulfonsäure und Toluolsulfonsäure. Bevorzugt sind Schwefelsäure und Salzsäure. Die Konzentration der Säure in wäßrig-saurem Reaktionsgemisch kann beispielsweise 5 bis 30 Gew.-% betragen. Vorzugsweise beträgt sie 10 bis 20 Gew.-%. Die Säuremenge kann beispielsweise 0,5 bis 10 Äquivalente Säure pro Äquivalent eingesetztes Nitrobenzol der Formel (II) betragen. Vorzugsweise beträgt diese Menge 1 bis 5 Äquivalente, insbesondere 1,2 bis 3 Äquivalente.

Bezogen auf das wäßrig-saure Reaktionsmedium kann man beispielsweise 5 bis 50 Gew.-%, vorzugsweise 10 bis 25 Gew.-% eines Nitrobenzols der Formel (II) einsetzen.

Gegebenenfalls kann man in zusätzlicher Gegenwart eines Co-Solvens arbeiten. Bei den Co-Solventien kann es sich um mit Wasser mischbare organische Lösungsmittel handeln, beispielsweise um wassermischbare cyclische oder offenkettige Ether wie Tetrahydrofuran, 1,4-Dioxan, Glykolmonoalkylether oder Glykoldialkylether, um wassermischbare Ester oder Amide wie Essigsäureethylester, N,N-Dimethylformamid, N,N-Dimethylacetamid oder N-Methylpyrrolidon, um niedere aliphatische Carbonsäuren wie Ameisensäure, Essigsäure oder Propionsäure, um wassermischbare Ketone wie Aceton oder Methylethylketon oder um wassermischbare Alkohole wie Methanol, Ethanol, Propanol oder Ethylenglykol. Bevorzugt sind Methanol, Ethanol, Ethylenglykol, Ethylenglykolmonomethylether, Ethylenglykoldimethylether und 1,4-Dioxan. Co-Solventien kann man gegebenenfalls in der 0,05- bis 3-fachen, vorzugsweise 0,1-bis 1-fachen Gewichtsmenge, bezogen auf das eingesetzte Nitrobenzol der Formel (II), einsetzen.

Zusätzlich zu dem Co-Solvens kann gegebenenfalls ein wasserunlösliches organisches Lösungsmittel eingesetzt werden, in dem das eingesetzte Nitrobenzol der Formel (II) zumindest teilweise löslich ist. Beispiele sind gegebenenfalls alkyl- und/oder halogen-substituierte aromatische Kohlenwasserstoffe wie Benzol, Toluol, Xylole, Chlorbenzol, Dichlorbenzole, Chlortoluole oder Dichlortoluole. Bevorzugt sind Toluol und Xylole. Wasserunlösliche organische Lösungsmittel kann man gegebenenfalls in der 0,1- bis 5-fachen, vorzugsweise 0,25- bis 3-fachen Gewichtsmenge, bezogen auf das eingesetzte Nitrobenzol der Formel (II) einsetzen.

Es ist bevorzugt in Gegenwart eines Co-Solvens und eines wasserunlöslichen Lösungsmittels zu arbeiten. Als Katalysatoren für die katalytische hydrierende Hydroxylierung kommen z.B. Edelmetalle und Edelmetallverbindungen der Platingruppen-Elemente in Frage, insbesondere Platin und/oder Palladium und/oder deren Verbindungen. Die Katalysatoren können gegebenenfalls auf einem Trägermaterial angeordnet sein. Als Trägermaterialien kommen z.B. Silikagele, Aluminiumoxide, Zeolithe, Molekularsiebe oder Kohlen in Frage, deren Belegung mit Edelmetallen oder Edelmetallverbindungen beispielsweise 0,05 bis 10 Gew.-%, vorzugsweise 0,1 bis 5 Gew.-% betragen kann. Bezogen auf das eingesetzte Nitrobenzol der Formel (II) kann man beispielsweise 0,001 bis 0,3 Gew.-%, vorzugsweise 0,01 bis 0,1 Gew.-% des Katalysators (gerechnet als Metall) einsetzen.

Die katalytische hydrierende Hydroxylierung kann man z.B. bei Temperaturen von 50 bis 160°C und Drucken von 1 bis 50 bar durchführen. Bevorzugt sind 60 bis 130°C und 1,2 bis 20 bar.

Es ist vorteilhaft, das Reaktionsgemisch während der Wasserstoffaufnahme gut zu durchmischen, beispielsweise durch die Anwendung von Rührern, Hubeinrichtungen oder Schüttelautoklaven.

Das nach Beendigung der katalytischen hydrierenden Hydroxylierung vorliegende Reaktionsgemisch kann man z.B. aufarbeiten, indem man zunächst den Katalysator, z.B. durch Filtration, abtrennt, dann die organische Phase abtrennt und entfernt, die wäßrige Phase mit einer Base versetzt und z.B. die sich bei einem pH-Wert von ungefähr 5 bis 8 bildende Suspension filtriert, den Filtrationsrückstand mit Wasser wäscht und trocknet. Man kann die wäßrige Phase zunächst auch mit so viel Base versetzen, bis das Gemisch stark alkalisch reagiert, dann unerwünschte Nebenprodukte durch Extraktion mit einem organischen Lösungsmittel entfernen und danach einen pH-Wert im Bereich von ungefähr 5 bis 8 einstellen. Als Basen kommen z.B. Hydroxide und Carbonate von Alkali- und Erdalkalimetallen in Frage, die gegebenenfalls auch als wäßrige Lösung oder Suspension eingesetzt werden können. Bevorzugt sind Natrium- und Kaliumhydroxid.

Das bei der katalytischen hydrierenden Hydroxylierung erhaltene freie Aminophenol der Formel (III) kann direkt in die 2. Stufe des erfindungsgemäßen Verfahrens, der Umsetzung mit einem halogenierten Olefin, eingesetzt werden.

Für diese Umsetzung kommen polare, aprotische Lösungsmittel beispielsweise Amide, Sulfone, Nitrile, Ketone und Ether in Frage. Beispiele sind Dimethylformamid, Dimethylacetamid, N-Methylpyrrolidon, Tetramethylensulfon, Acetonitril, Propionitril, Aceton, Methylethylketon, Tetrahydrofuran und 1,4-Dioxan. Besonders bevorzugt ist Acetonitril.

Polare, aprotische Lösungsmittel können beispielsweise in Mengen von 200 bis 2 000 ml, bezogen auf 1 mol Aminophenol der Formel (III), eingesetzt werden. Vorzugsweise liegt diese Menge bei 500 bis 1 000 ml

Für die Umsetzung von freien Aminophenolen der Formel (III) mit halogenierten Olefinen kommen als Basen z.B. Hydroxide, Carbonate und Hydrogencarbonate von Alkali- und Erdalkalimetallen, Ammoniak und organische Amine in Frage. Beispiele sind Natrium-, Natriumhydrogen-, Kalium-, Cäsium- und Ammoniumcarbonat, Lithium-, Natrium-, Kalium- und Ammoniumhydroxid, Triethyl- und Tributylamin, Pyridin, 4-Dimethylaminopyridin und DBU.

Bevorzugt sind Natriumcarbonat, Triethylamin und Alkalihydroxide.

Die Base kann beispielsweise in einer Menge eingesetzt werden, die 1 bis 50 Gew.-%, bezogen auf das freie Aminophenol der Formel (III), entspricht. Man kann die Base auch während der Reaktion so zudosieren, daß stets der pH-Wert des Reaktionsgemisches im Bereich von 6 bis 9,5 verbleibt.

Das Reaktionsgemisch für die Umsetzung eines freien Aminophenols der Formel (III) mit einem halogenierten Olefin kann beispielsweise 0,5 bis 30 Gew.-% Wasser enthalten, wobei das Wasser z.B. vorgelegt und/oder zusammen mit der Base vor und/oder kontinuierlich in kleinen Portionen während der Reaktion zugesetzt werden kann.

Das halogenierte Olefin wird vorzugsweise während der Reaktion entsprechend dem Verbrauch kontinuierlich oder in kleinen Portionen zugeführt. Das Fortschreiten der Reaktion kann man z.B. am Druckabfall erkennen, insbesondere wenn man die Reaktion bei erhöhtem Druck durchführt.

Bevorzugt ist es, die Base in Form einer wäßrigen Lösung und das halogenierte Olefin gleichzeitig, aber räumlich getrennt während der Reaktion kontinuierlich oder in kleinen Portionen zuzuführen.

Als Temperaturen für die Umsetzung eines freien Aminophenols der Formel (III) mit einem halogenierten Olefin kommen z.B. solche im Bereich 0 bis 120°C in Frage.

Bevorzugt sind 5 bis 30°C. Der Druck bei dieser Reaktion kann z.B. im Bereich von 0,5 bis 5 bar liegen. Bevorzugt liegt er bei 1 bis 2,5 bar.

Man erhält p-Haloalkoxyaniline der Formel (I) in besonders guten Ausbeuten und Reinheiten, wenn man bei relativ niedriger Temperatur arbeitet und die Base simultan mit dem halogenierten Olefin im Verlauf der Reaktion zudosiert, und zwar in dem Maße, wie das halogenierte Olefin verbraucht wird.

Das nach der Umsetzung des freien Aminophenols der Formel (III) mit dem halogenierten Olefin vorliegende Reaktionsgemisch kann man z.B. durch Destillation oder Extraktion aufarbeiten. Bevorzugt ist die Destillation. Man kann dabei das polare, aprotische Lösungsmittel abtrennen und gegebenenfalls erneut einsetzen und anschließend das hergestellte p-Halogenalkoxyanilin der Formel (I) in guten Ausbeuten und Reinheiten isolieren. Vorzugsweise führt man die Destillation des Produkts im Vakuum durch, z.B. bei 1 bis 20 mbar.

Das erfindungsgemäße Verfahren umfaßt nur zwei Reaktionsstufen, geht von gut zugänglichen Edukten aus und benötigt keinen besonderen Verfahrensaufwand.

### Beispiele

### Beispiel 1

In einem Emailautoklaven wurden 480 g 2,5-Dichlornitrobenzol, 2000 g Wasser, 306 g konzentrierte Schwefelsäure, 85 g 1,2-Dimethoxyethan, 4,8 g 5 Gew.-% Platinauf-Kohle (ca. 60 Gew.-% feucht) und 417 g Toluol bei Raumtemperatur vorgelegt. Nach dem Inertisieren mit Stickstoff wurde unter Rühren bei 105°C und 9 bis 9,5 bar Wasserstoff eingeleitet. Nach dem Erreichen der Druckkonstanz wurde entspannt und der Katalysator durch Filtrieren in der Hitze entfernt. Die wäßrige Phase wurde heiß abgetrennt, mit 50 gew.-%iger wäßriger Natronlauge auf einen pH-Wert von 12 eingestellt, mit 500 ml Toluol extrahiert und anschließend mit konzentrierter Salzsäure auf einen pH-Wert von 6 eingestellt. Die anfallende Suspension wurde bei Raumtemperatur abfiltriert und mit Wasser gewaschen. Nach dem Trocknen im Vakuum verblieben 273,6 g eines bräunlich-rötlichen Feststoffs. Dessen Gehalt an 2,5-Dichlor-4-aminophenol betrug 98 %. Das entsprach einer Ausbeute von 60,3 %.

### Beispiel 2

In einem Reaktionsgefäß mit Rührer, Rückflußkühler und Einleitungsrohr wurden 80 g 2,5-Dichlor-4-aminophenol gelöst in 400 ml Acetonitril vorgelegt und anschließend 9 ml gesättigte Sodalösung zudosiert. In die entstandene Suspension wurde unter kräftigem Rühren Hexafluorpropen im Maße der Aufnahme eingeleitet. Die Reaktion war leicht exotherm, wodurch die Temperatur von 22°C auf 30°C anstieg. Nach 2 Stunden wurden nochmals 5 ml der Sodalösung zudosiert und weiter Hexafluorpropen eingeleitet. Nach insgesamt 3 Stunden lag die Temperatur bei 32°C, es war eine fast klare Lösung entstanden und die Aufnahme von Hexafluorpropen zum Ende gekommen. Der Verbrauch an Hexafluorpropen betrug 80 g. Der pH-Wert der Lösung lag nunmehr bei 5. Danach wurde durch Temperaturerhöhung das Acetonitril abdestilliert und anschließend Vakuum angelegt. Im Vorlauf gingen 380 ml Acetonitril über. Nach einem kleinen Zwischenlauf wurden bei 145°C und 20 mbar 137 g 2-H-Hexafluorpropoxy-2,5-dichloranilin erhalten. Die Reinheit (gaschromatografisch bestimmt) betrug 97 %. Als hauptsächliche Nebenkomponente waren 1,5 % eines durch Eliminierung von Fluorwasserstoff entstandenen Produktes enthalten. Die Ausbeute betrug 90 %, bezogen auf 100 % Gehalt von Ausgangsmaterial und Produkt.

### Beispiel 3

In einem Autoklaven mit Rührer, Rückflußkühler und Einleitungsrohr wurden 160 g 2,5-Dichlor-4-aminophenol gelöst in 700 ml Acetonitril vorgelegt und anschließend 10 ml Triethylamin und 10 ml Wasser zudosiert. In die entstehende Mischung wurde bei 20°C unter kräftigem Rühren portionsweise Hexafluorpropen eingeleitet. Die Reaktion war leicht exotherm, wodurch die Temperatur auf 25°C anstieg. Nach 5 Takten waren insgesamt 150 g Hexafluorpropen eindosiert. Nach insgesamt 3 Stunden Reaktionszeit wurde die Reaktionsmischung in eine Destillationsapparatur übergeführt und durch Temperatursteigerung das Acetonitril abdestilliert. Es gingen im Vorlauf 684 ml Acetonitril über. Nach einem kleinen Zwischenlauf wurden im Siedebereich von 145 bis 148°C und 18 mbar 275 g 2-H-Hexafluorpropoxy-2,5-dichloranilin erhalten. Die Reinheit des Produktes (gaschromatografisch bestimmt) betrug 97,2 %. Die wesentlichen Nebenkomponenten waren mit 2,75 % Fluorwasserstoff-Eliminierungsprodukte. Die Ausbeute betrug demnach 90,3 %, bezogen auf eingesetztes Aminophenol und 83,8 %, bezogen auf Hexafluorpropen.

### Beispiel 4 (zum Vergleich)

Es wurde gearbeitet wie in Beispiel 2 jedoch wurde statt Acetonitril weiteres Wasser eingesetzt. Auf diese Weise wurde 2-H-Hexafluorpropoxy-2,5-dichloranilin in einer Ausbeute von nur 10% erhalten.

## Patentansprüche

1. Verfahren zur Herstellung von p-Haloalkoxyanilinen der Formel (I) 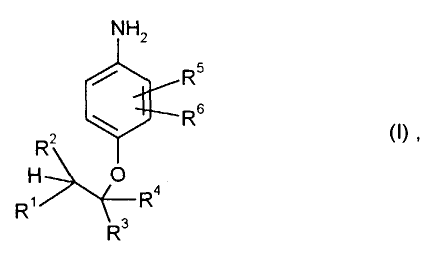 in der
R¹ Wasserstoff Halogen oder C₁-C₄-Halogenalkyl und
R² bis R⁶ unabhängig voneinander jeweils Wasserstoff oder Halogen bedeuten, wobei mindestens einer der Reste R¹ bis R⁴ verschieden von Wasserstoff ist,
dadurch gekennzeichnet, daß man ein Nitrobenzol der Formel (II) 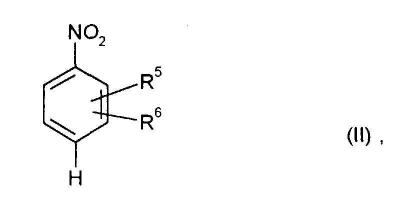 in der
R⁵ und R⁶ die bei Formel (I) angegebene Bedeutung haben,
durch katalytische hydrierende Hydroxylierung in Gegenwart eines wäßrigsauren Reaktionsmediums in ein freies Aminophenol der Formel (III) umwandelt 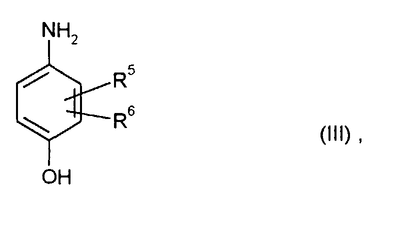 in der
R⁵ und R⁶ die bei Formel (I) angegebene Bedeutung haben,
und dieses Aminophenol mit einem halogenierten Olefin der Formeln (IV) oder (V) 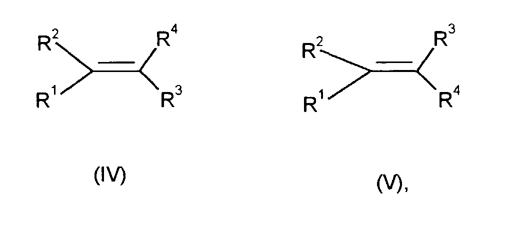 in denen
R¹ bis R⁴ die bei Formel (I) angegebene Bedeutung haben,
in Gegenwart von Wasser und einer katalytischen Menge an Base umsetzt.

2. Verfahren nach Anspruch 1, dadurch gekennzeichnet, daß in den Formeln R¹ für C₁-C₂-Halogenalkyl, Fluor oder Chlor, R² bis R⁴ unabhängig voneinander für Wasserstoff, Fluor oder Chlor und R⁵ und R⁶ unabhängig voneinander für Wasserstoff, Fluor, Chlor oder Brom stehen, wobei mindestens einer der Reste R⁵ und R⁶ nicht Wasserstoff ist.

3. Verfahren nach Ansprüchen 1 und 2, dadurch gekennzeichnet, daß das wäßrig-saure Reaktionsmedium aus einem Gemisch besteht, das Wasser und eine starke Säure enthält.

4. Verfahren nach Ansprüchen 1 bis 3, dadurch gekennzeichnet, daß die Konzentration der Säure im wäßrig-sauren Reaktionsgemisch 5 bis 30 Gew.-% und die Säuremenge 0,5 bis 10 Äquivalente Säure pro Äquivalent eingesetztes Nitrobenzol der Formel (II) beträgt.

5. Verfahren nach Ansprüchen 1 bis 4, dadurch gekennzeichnet, daß man die katalytische hydrierende Hydroxylierung in zusätzlicher Gegenwart eines Co-Solvens durchführt, bei dem es sich um ein mit Wasser mischbares organisches Lösungsmittel handelt.

6. Verfahren nach Ansprüchen 1 bis 5, dadurch gekennzeichnet, daß man die katalytische hydrierende Hydroxylierung in Gegenwart eines Co-Solvens durchführt, bei dem es sich um ein mit Wasser mischbares organisches Lösungsmittel handelt und in zusätzlicher Gegenwart eines wasserunlöslichen organischen Lösungsmittels.

7. Verfahren nach Ansprüchen 1 bis 6, dadurch gekennzeichnet, daß man die katalytische hydrierende Hydroxylierung bei 50 bis 160°C und 1 bis 50 bar durchführt.

8. Verfahren nach Ansprüchen 1 bis 7, dadurch gekennzeichnet, daß man die Umsetzung mit einem halogenierten Olefin in Gegenwart von Hydroxiden, Carbonaten oder Hydrogencarbonaten von Alkali- oder Erdalkalimetallen oder Ammoniak oder organischen Aminen durchführt.

9. Verfahren nach Ansprüchen 1 bis 8, dadurch gekennzeichnet, daß man bei der Umsetzung mit halogenierten Olefinen 1 bis 50 Gew.-% Base einsetzt (bezogen auf das freie Aminophenol der Formel (III)).

10. Verfahren nach Ansprüchen 1 bis 9, dadurch gekennzeichnet, daß das Reaktionsgemisch für die Umsetzung mit einem halogenierten Olefin 0,5 bis 30 Gew.-% Wasser enthält und man bei 0 bis 120°C und 0,5 bis 5 bar arbeitet.
